# EUROPÄISCHE PATENTSCHRIFT

(11) **EP 1 049 494 B1**
(45) Veröffentlichungstag und Bekanntmachung des Hinweises auf die Patenterteilung: **13.11.2002**
(21) Anmeldenummer: 98962176.8
(22) Anmeldetag: 22.10.1998
(51) Int. Cl.: A61M 1/00, G10L 15/26

(54) **VERWENDUNG EINER SPRACHEINGABEEINRICHTUNG IN EINEM BLUTENTNAHMEGERÄT**
USE OF SPEECH INPUT DEVICE IN A BLOOD WITHDRAWAL DEVICE
UTILISATION D'UN DISPOSITIF POUR L'ENTREE VOCALE POUR UN APPAREIL DE PRELEVEMENT DE SANG

(30) Priorität: 18.11.1997 DE 19751072
(43) Veröffentlichungstag der Anmeldung: 08.11.2000
(73) Patentinhaber: LMB Technologie GmbH, 85445 Schwaig (DE)
(72) Erfinder: JENTSCH, Klaus, Dieter, D-85445 Schwaig (DE)
(74) Vertreter: Winter, Brandl, Fürniss, Hübner, Röss, Kaiser, Polte Partnerschaft
(86) Internationale Anmeldenummer: DE9803109
(87) Internationale Veröffentlichungsnummer: WO99025397

(56) Entgegenhaltungen:
- EP-A- 0 733 377
- EP-A- 0 813 171
- WO-A-97/31345
- DE-U- 29 611 131
- US-A- 4 605 080
- US-A- 5 126 543
- US-A- 5 389 917

## Beschreibung

Die vorliegende Erfindung betrifft die Verwendung einer Spracheingabeeinrichtung in einem Blutentnahmegerät, und zwar insbesondere in einem solchen Blutentnahmegerät, das bei der Blutspende, Eigenblutspende oder Blutkomponentenspende angewendet wird. Zu derartigen Blutentnahmegeräten zählen insbesondere solche Geräte wie Blutentnahmemonitore, Blutentnahmewaagen, Blutmischmonitore, Blutwaagen, Blutmonitore, Mischwaagen, Blutmixer, usw.

Aus der **EP-A-0 733 377** ist das in Fig. 5 gezeigte Blutentnahmegerät bekannt, von dem die Erfindung ausgeht und bei dem eine Spracheingabeeinrichtung nach der Lehre der vorliegenden Erfindung verwendbar ist. Dieses bekannte Blutentnahmegerät ist allgemein mit dem Bezugszeichen 10 bezeichnet und weist eine Aufnahmevorrichtung 20, auf welche bei der Blutentnahme ein Blutbeutel (nicht gezeigt) gelegt ist, eine Zufuhrstoppvorrichtung 30, ein Tastenfeld 40 sowie ein Gehäuse 50 auf.

Wenn eine Blutentnahme, zum Beispiel bei einer Blutspende, Eigenblutspende oder Blutkomponentenspende, durchzuführen ist, wird ein Blutbeutel, der das entnommene Blut aufnimmt, auf die Aufnahmevorrichtung 20 des Blutentnahmegeräts 10 gelegt und wird ein an dem Blutbeutel vorgesehener Entnahmeschlauch (nicht gezeigt) in die Zufuhrstoppvorrichtung 30 eingebracht. Wenn nun Blut über den Entnahmeschlauch in den Blutbeutel fließt, wird die Menge des in dem Blutbeutel enthaltenen Bluts mittels einer in dem Gehäuse 50 vorgesehenen Wägeeinrichtung ermittelt, die mit der Aufnahmevorrichtung 20 gekoppelt ist. Wenn eine bestimmte Blutmenge in dem Beutel enthalten ist, wird die Zufuhrstoppvorrichtung 30 betätigt, wodurch die Blutzufuhr durch den Entnahmeschlauch zu dem Blutbeutel gestoppt wird. Zum Beispiel kann die Zufuhrstoppvorrichtung 30 eine Klemmvorrichtung sein, die elektrisch betätigt wird und den Entnahmeschlauch bei Betätigung abklemmt. Mittels des Tastenfelds 40 können für das Blutentnahmegerät 10 wichtige Einstellungen vorgenommen werden. So kann zum Beispiel ein wert einer zu entnehmenden Blutmenge festgelegt werden.

Weiterhin ist in dem Gehäuse eine Bewegungseinrichtung vorgesehen, die die Aufnahmevorrichtung 20 schwenkend bewegt, um in dem Blutbeutel vorhandenes Blut mit einem ebenfalls in dem Blutbeutel vorhandenen Stabilisator zu vermischen und so eine Gerinnung des Bluts zu verhindern.

Bei der Blutentnahme ist es erforderlich, zahlreiche zusätzliche Daten zu erfassen, wobei diese Daten in für ein jeweiliges Land gültigen Richtlinien festgelegt sind. Diese jeweils zu erfassenden Daten beinhalten z.B. folgende Werte: Blutdruck, Hematokrit/Hemaglobin, verantwortlicher Arzt, sonstige Daten einer Gesundheitsprüfung, Entnahmepersonal, Spendername, Spendergeburtsdatum, Spenderwohnort, Spendertelefonnummer, besondere Merkmale des Spenders, Datum der Spende, Uhrzeit der Spende, Dauer der Spende, sonstige Maschinendaten, Blutkonservennummer, Artikelnummer des Blutbeutels, Identifikationsnummer des Blutbeutels, Chargennummer des Beutelsystems, Aufzeichnung von Verlaufsproblemsn, usw..

Diese zusätzlichen Daten sind bisher per Hand oder maschinell auf Etiketten aufgebracht worden, die auf die Blutbeutel geklebt worden sind oder sind in den Begleitpapieren vermerkt worden. Wenn die Daten per Hand vermerkt werden, besteht jedoch die Gefahr eines falschen Lesens der Daten aufgrund einer undeutlichen Handschrift.

Weiterhin sind Blutentnahmegeräte bekannt, die ein Eingeben von Daten in das Blutentnahmegerät, mittels einer Tastatur oder eines Strichcodelesers ermöglichen. Bei der Dateneingabe mittels Tastatur ist es jedoch für einen schnellen Ablauf der Dateneingabe erforderlich, daß der Bediener einen qualifizierten Umgang mit der Tastatur beherrscht, was jedoch was jedoch nicht immer der Fall ist. Weiterhin besteht die Möglichkeit von Schreibfehlern, die sich insbesondere bei derartigen Daten, wie zum Beispiel der Blutgruppe, äußerst negativ auswirken können, wenn sie nicht bemerkt und verbessert werden. Bei der Dateneingabe mittels Strichcode können lediglich Daten erfaßt werden, die in einem Strichcodeformat vorliegen, wie es zum Beispiel bei der Artikelnummer oder Identifikationsnummer des Blutbeutels der Fall sein kann, wenn diese im Strichcodeformat auf dem Blutbeutel vorgesehen ist. Jedoch setzt eine fehlerfreie Erkennung mittels des Strichcodeslesers voraus, daß der zu erfassende Strichcode fehlerfrei zu lesen ist, was zum Beispiel bei Kratzern oder einer Verunreinigung auf dem Strichcode nicht der Fall ist.

Aus der **DE-U-296 11 131** ist ein weiteres Blutentnahmegerät bekannt, bei dem zusätzlich eine Übertragungseinrichtung zum drahtlosen wechselseitigen Übertragen von Daten mit einer externen Verarbeitungseinheit vorgesehen ist, wobei mehrere derartige Blutentnahmegeräte und die externe Verarbeitungseinheit zu einem System gekoppelt sein können.

Aus der **US-A-4 605 080** ist eine Wägeeinrichtung bekannt, die ein Spracherkennungs-Steuersystem aufweist, mit dem die Wägeeinrichtung gesteuert werden kann und mit dem zum Beispiel weitere Vorgänge eingeleitet und durchgeführt werden können. In dieser Druckschrift wird jedoch nicht in Erwägung gezogen, das Spracherkennungs-Steuersystems auch zur Speicherung von zusätzlichen Daten zu verwenden, die nicht die Ansteuerung der wägeeinrichtung selbst betreffen,. Darüber hinaus ist diese bekannte Wägeeinrichtung ausschließlich zur Verwendung in einem automatisierten Ablauf zum fortlaufenden Abwiegen und Etikettieren von Massengütern vorgesehen.

Die Aufgabe der vorliegenden Erfindung besteht darin, ein Blutentnahmegerät der gattungsgemäßen Art so weiterzibilden, daß eine beliebig große Anzahl unterschiedlicher Daten mit großer Zuverlässigkeit eingegeben werden können, so daß letztendlich die Handhabung eines solchen Blutentnahmegeräts wesentlich vereinfacht wird.

Diese Aufgabe wird erfindungsgemäß daduruch gelöst, daß in einem gattungsgemäßen Blutentnahmegerät eine Spracheingabeeinrichtung verwendet wird, die diejenigen Daten erfaßt, die in für ein jeweiliges Land gültigen Richtlinien zur Blutentnahme festgelegt sind, wobei die auf diese Weise erfaßten, zunächst in einem Sprachformat vorliegenden Daten anschließend mittels einer Wandlereinrichtung in solche Daten umgewandelt werden, die von dem Blutentnahmegerät verarbeitet werden können.

Durch die erfindungsgemäße Art der Eingabe der bei der Blutentnahme relevanten bzw. gesetzlich festgelegten Daten wird einerseits erreicht, daß diese Daten wesentlich flexibler und schneller erfaßt werden können, während andererseits Fehleingaben relativ sicher verhindert werden können, so daß die spätere Zuordnung bzw. Auswertung der Daten des entnommenen Bluts deutlich zuverlässiger durchgeführt werden kann.

Weitere vorteilhafte Ausgestaltungen der vorliegenden Erfindung sind Gegenstand der Unteransprüche.

Die vorliegende Erfindung wird nachstehend anhand der Beschreibung von Ausführungsbeispielen unter Bezugnahme auf die beiliegende Zeichnung näher erläutert.

Es zeigt:
- **Fig. 1**: eine schematische Darstellung der Verwendung einer Spracheingabeeinrichtung in einem Blutentnahmegerät gemäß einem ersten Ausführungsbeispiel der vorliegenden Erfindung;
- **Fig. 2**: eine schematische Darstellung der Verwendung einer Spracheingabeeinrichtung in einem Blutentnahmegerät gemäß einem zweiten Ausführungsbeispiel der vorliegenden Erfindung;
- **Fig. 3**: eine schematische Darstellung der Verwendung einer Spracheingabeeinrichtung in einem Blutentnahmegerät gemäß einem dritten Ausführungsbeispiel der vorliegenden Erfindung;
- **Fig. 4**: eine schematische Darstellung eines mehrere erfindungsgemäße Blutentnahmegeräte verwendenden Systems gemäß einem vierten Ausführungsbeispiel der vorliegenden Erfindung? und
- **Fig. 5**: eine perspektivische Ansicht eines Blutentnahmegeräts im Stand der Technik.

Es folgt die Beschreibung von Ausführungsbeispielen der vorliegenden Erfindung.

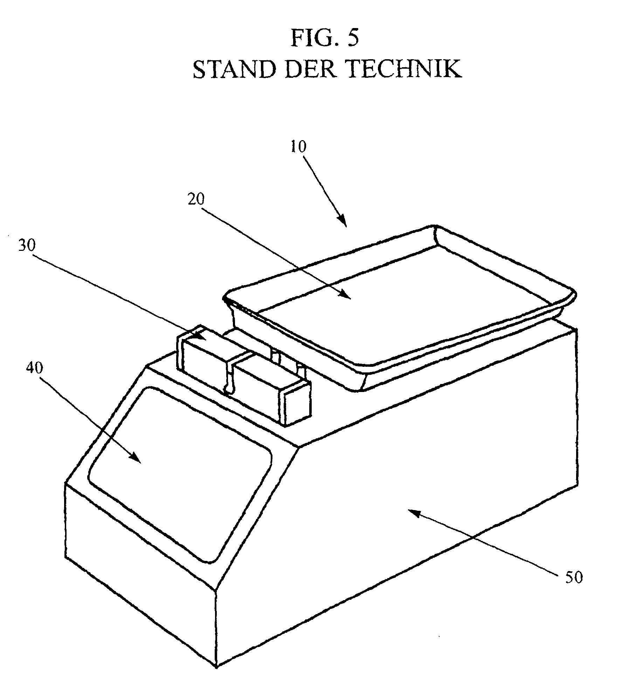

Es ist anzumerken, daß durchgängig durch die Ausführungsbeispiele das erfindungagemäße Blutentnahmegerät ein zu dem in Fig. 5 gezeigten Blutentnahmegerät im Stand der Technik ähnliches Erscheinungsbild aufweist. Aus diesem Grund sind in den die Ausführungsbeispiele betreffenden Figuren lediglich schematische Darstellungen des erfindungsgemäßen Blutentnahmegeräts gezeigt.

Nachstehend erfolgt die Beschreibung eines ersten Ausführungsbeispiels der vorliegenden Erfindung.

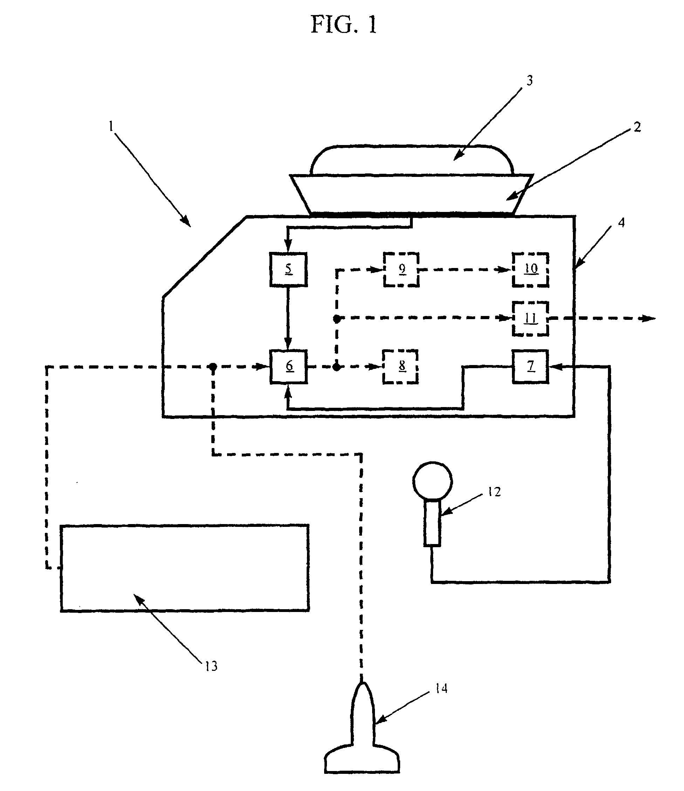

Fig. 1 zeigt eine schematische Darstellung eines Blutentnahmegeräts gemäß dem ersten Ausführungsbeispiel der vorliegenden Erfindung. Vorzugsweise ist dieses Blutentnahmegerät 1 tragbar, so daß es ortsbeweglich eingesetzt werden kann. Dieses Blutentnahmegerät 1 weist eine Aufnahmevorrichtung 2 auf, die einen Blutbeutel bzw. ein Blutentnahmegefäß 3 aufnimmt. Weiterhin sind eine Bewegungseinrichtung sowie eine Zufuhrstoppvorrichtung (beides nicht gezeigt) vorgesehen. In einem Gehäuse 4 des Blutentnahmegeräts 1 sind eine Wägeeinrichtung 5, eine Erfassungseinrichtung 6, eine Wandlereinrichtung 7, eine Ausgabeeinrichtung 8, eine Verarbeitungseinrichtung 9, eine Speichereinrichtung 10 sowie eine Übertragungseinrichtung 11 untergebracht. An die Wandlereinrichtung 7 ist ein als eine Spracheingabeeinrichtung dienendes Mikrofon 12 gekoppelt, das zum Beispiel getrennt von dem Blutentnahmegerät 1 vorgesehen sein kann. Alternativ kann das Mikrofon 12 in das Blutentnahmegerät 1 eingebaut vorgesehen sein. Weiterhin sind an die Erfassungseinrichtung 6 eine vorzugsweise externe Tastatur 13 sowie ein Strichcodeleser 14 gekoppelt.

Die Funktionsweise dieses Blutentnahmegeräts 1 ist wie folgt.

Vor einer Blutentnahme wird der Blutbeutel 3 auf die Aufnahmevorrichtung 2 gelegt. Ein Entnahmeschlauch (nicht gezeigt) wird in die Zufuhrstoppvorrichtung eingebracht. Diese Zufuhrstoppeinrichtung kann zum Beispiel eine Klemmvorrichtung sein, die eine Blutzufuhr in den Blutbeutel durch Abklemmen des Entnahmeschlauchs stoppt. Die an die Aufnahmevorrichtung 2 gekoppelte Wägeeinrichtung 5 wägt einen Inhalt des Blutbeutels 3 und bestimmt somit eine in dem Blutbeutel 3 enthaltene Menge. Daten, die diese Menge anzeigen, werden aus der Wägeeinrichtung 5 zu der Erfassungseinrichtung 6 ausgegeben. Wenn eine entnommene Blutmenge eine vorbestimmte Menge erreicht hat, wird die Zufuhrstoppvorrichtung betätigt, um die Blutzufuhr durch den Entnahmeschlauch zu dem Blutbeutel 3 automatisch zu stoppen. Die Aufnahmevorrichtung kann mittels der Bewegungseinrichtung in eine Schwenkbewegung versetzt werden, um dadurch das in dem Blutbeutel 3 enthaltene Blut mit einem ebenso in dem Blutbeutel 3 vorhandenen Stabilisator zu vermischen und eine Gerinnung des Bluts in dem Blutbeutel 3 zu verhindern.

Daten, die für eine jeweilige Blutentnahme erforderlich sind, welche je nach in einzelnen Ländern gültigen Richtlinien unterschiedlich sind, beinhalten: Blutdruck, Hematokrit/Hemaglobin, verantwortlicher Arzt, sonstige Daten einer Gesundheitsprüfung, Entnahmepersonal, Spendername, Spendergeburtsdatum, Spenderwohnort, Spendertelefonnummer, besondere Merkmale des Spenders, Datum der Spende, Uhrzeit der Spende, Dauer der Spende, sonstige Maschinendaten, Blutkonservennummer, Artikelnummer des Blutbeutels, Identifikationsnummer des Blutbeutels, Chargennummer des Beutelsystems, Aufzeichnung von Verlaufsproblemen, usw.. Diese Daten werden wie folgt in das Blutentnahmegerät 1 eingegeben.

Wie es vorhergehend beschrieben worden ist, sind das als Spracheingabeeinrichtung dienende Mikrofon 12, die Tastatur 13 und der Strichcodeleser 14 an das Blutentnahmegerät 1 gekoppelt. Daten, die im Strichcodeformat vorliegen, können somit mittels des Strichcodelesers 14 in das Blutentnahmegerät 1 eingegeben werden. Daten, die nicht im Strichcodeformat vorliegen, können sowohl mittels der Tastatur 13 als auch insbesondere des Mikrofons 12 eingegeben werden. Insbesondere bei Verwendung des Mikrofons 12 zur Dateneingabe mittels Sprache wird eine hohe Zuverlässigkeit bei der Dateneingabe erzielt, da Schreibfehler, wie sie bei der Tastatur 13 vorkommen können, vermieden werden. Ferner wird die Bedienung des Blutentnahmegeräts 1 durch den Bediener wesentlich vereinfacht, da die Tastatur 13 nicht zwingend verwendet werden muß. Die Dateneingabe kann weiterhin sowohl entweder durch die Tastatur 13, den Strichcodeleser 14 oder das Mikrofon 12 als auch durch jede mögliche Kombination dieser Eingabeeinrichtungen erfolgen, was eine hohe Bedienflexibilität des Blutentnahmegeräts 1 sicherstellt.

Die von der Tastatur 13 und dem Strichcodeleser 14 eingegeben Daten werden in die Erfassungseinrichtung 6 eingegeben und die von dem Mikrofon 12 eingegebenen Daten werden in die Wandlereinrichtung 7 eingegeben. Die Wandlereinrichtung 7 wandelt die eingegebenen Sprachdaten in Daten eines verarbeitbaren Datenformats und gibt diese Daten zu der Erfassungseinrichtung 6 aus. Somit nimmt die Erfassungseinrichtung 6 Daten von der Wägeeinrichtung 5, von der Tastatur 13, von dem Strichcodeleser 14 als auch von der Wandlereinrichtung 7 auf. Die Erfassungseinrichtung 6 gibt die erfaßten Daten zu der Ausgabeeinrichtung 8 aus. Die Ausgabeeinrichtung 8 gibt die Daten derart aus, daß sie der Bediener kontrollieren kann und sie gegebenenfalls ebenso korrigieren kann. Diese Ausgabe der Daten kann zum Beispiel entweder durch eine optische Anzeige (mit Text, Ziffern, Graphik, usw.) an dem Blutentnahmegerät 1 oder durch eine Sprachausgabe der Daten mittels eines als Sprachausgabeeinrichtung dienenden Lautsprechers oder ähnlichem erfolgen.

Weiterhin werden die erfaßten Daten von der Verarbeitungseinrichtung 9 zweckmäßig verarbeitet bzw. aufbereitet. Die verarbeiteten Daten werden dann zu der Speichereinrichtung 10 ausgegeben und in dieser gespeichert. Schließlich besteht ebenso die Möglichkeit, die Daten durch die Übertragungseinrichtung 11 zu einer externen Verarbeitungseinheit (nicht gezeigt) zu übertragen.

Somit ist die prinzipielle Struktur des Blutentnahmegeräts gemäß dem ersten Ausführungsbeispiel beschrieben worden. Jedoch sind zahlreiche Ausgestaltungen dieses Ausführungsbeispiels möglich.

So kann die Übertragungseinrichtung sowohl eine drahtgebundene Übertragungseinrichtung als auch eine drahtlose Übertragungseinrichtung sein. Zum Beispiel kann die Übertragungseinrichtung eine RS232-Schnittstelle, eine Funkschnittstelle, eine Infrarotschnittstelle oder dergleichen sein. Insbesondere, wenn die Schnittstelle eine drahtlose Schnittstelle ist, besteht die Möglichkeit, eine einfache Kopplung des Blutentnahmegeräts mit einer externen Verarbeitungseinheit sicherzustellen, die die von dem Blutentnahmegerät zu ihr übertragenen Daten zweckmäßig weiterverarbeiten und speichern kann. Weiterhin ist es möglich, Daten von einer externen Verarbeitungseinheit zu dem Blutentnahmegerät zu übertragen. Dies kann durchgeführt werden, um zum Beispiel Daten eines bereits bekannten Spenders erneut zu verwenden, ohne diese neu einzugeben, und lediglich die bei der vorliegenden Blutentnahme entstehenden unterschiedlichen Daten neu einzugeben.

Weiterhin kann die Speichereinrichtung zum Beispiel eine abnehmbare Speichereinrichtung, wie zum Beispiel eine Speicherkarte, sein. Ein Beispiel einer derartigen Speicherkarte ist eine SRAM-Karte. Diese Karte kann von dem Blutentnahmegerät abgenommen werden und zur weiteren Verarbeitung der darin gespeicherten Daten in einen passenden Einschub einer externen Verarbeitungseinheit gesteckt werden. Die externe Verarbeitungseinheit kann dann die verarbeiteten Daten speichern.

Ferner kann das Blutentnahmegerät weiterhin eine Massenspeichereinrichtung aufweisen. Diese Massenspeichereinrichtung kann sowohl zum Speichern großer Datenmengen als auch zur Eingabe notwendiger Daten und Programme in das Blutentnahmegerät dienen. Ein Beispiel einer solchen Massenspeichereinrichtung ist ein CDROM-Laufwerk. Diese Massenspeichereinrichtung kann innerhalb oder außerhalb des Blutentnahmegeräts vorgesehen sein.

Zusätzliche Bedienelemente können an dem Blutentnahmegerät vorgesehen sein. Mit diesen Bedienelementen können zum Beispiel notwendige Einstellungen durchgeführt werden. Zum Beispiel kann mit einem derartigen Bedienelement die zu entnehmende Blutmenge im voraus eingestellt werden.

Die in dem Blutentnahmegerät enthaltenen Einrichtungen können vorzugsweise mittels eines mikrocomputergesteuerten Systems und unter Verwendung eines Mikrocomputers realisiert sein. Dieses System kann weiterhin weitere Einrichtungen enthalten, mittels denen weitere Daten, wie zum Beispiel eine Durchflußmenge des Bluts, direkt oder indirekt (durch Berechnen) erfaßt werden können.

Schließlich ist es nicht zwingend erforderlich, daß alle zuvor beschriebenen Bestandteile in dem Blutentnahmegerät enthalten sind, wie es durch gestrichelte Linien in Fig. 1 gezeigt ist. In einer einfachsten Ausgestaltung beinhaltet das Blutentnahmegerät lediglich die Aufnahmevorrichtung, die Wägeeinrichtung, die Spracheingabeeinrichtung, die Wandlereinrichtung sowie die Erfassungseinrichtung. Alle anderen Bestandteile können je nach erwünschtem Anwendungsumfang oder erwünschter Leistungsfähigkeit vorhanden sein oder nicht.

Gemäß dem ersten Ausführungsbeispiel der vorliegenden Erfindung ist es mittels der vorgesehenen Spracheingabeeinrichtung möglich, eine große Vielzahl und Vielfalt unterschiedlicher Daten mit einer hohen Zuverlässigkeit in das Blutentnahmegerät einzugeben. Weiterhin weist das Blutentnahmegerät eine einfache Handhabung auf, da die Spracheingabeeinrichtung vorgesehen ist. Mittels der wahlweise vorsehbaren Einrichtungen wird ferner ein leistungsfähiges Blutentnahmegerät erzielt, das unterschiedliche alternative Bedienweisen aufweist und das ferner mit einer externen Verarbeitungseinheit gekoppelt werden kann.

Nachstehend erfolgt die Beschreibung eines zweiten Ausführungsbeispiels der vorliegenden Erfindung.

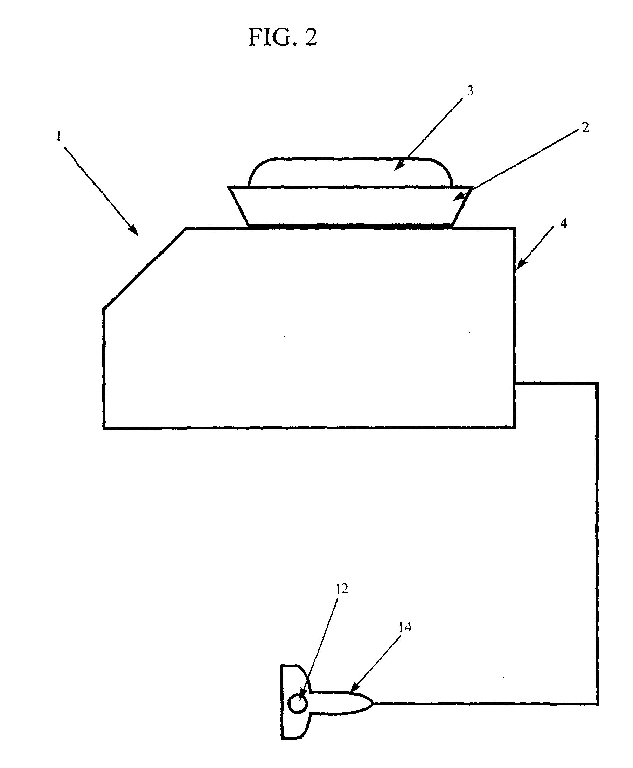

Fig. 2 zeigt eine schematische Darstellung des zweiten Ausführungsbeispiels der vorliegenden Erfindung. Dieses zweite Ausführungsbeispiel der vorliegenden Erfindung ist ausgenommen der nachstehend dargelegten Unterschiede zu ihrem ersten Ausführungsbeispiel gleich. Aus diesem Grund sind in Fig. 2 nur die zu dem ersten Ausführungsbeispiel unterschiedlichen Bestandteile dargestellt.

Gemäß dem zweiten Ausführungsbeispiel der vorliegenden Erfindung ist das als Spracheingabeeinrichtung dienende Mikrofon 12 nicht getrennt an das Blutentnahmegerät 1 gekoppelt, sondern ist in den Strichcodeleser 14 eingebaut vorgesehen. Damit kann die Bedienbarkeit des Blutentnahmegeräts 1 weiter vereinfacht werden. Alternativ kann das Mikrofon 12 an den Strichcodeleser 14 gekoppelt sein.

Ausgenommen dieses Unterschieds gelten alle anderen bezüglich des ersten Ausführungsbeispiels dargelegten Erläuterungen ebenso für das zweite Ausführungsbeispiel.

Nachstehend erfolgt die Beschreibung eines dritten Ausführungsbeispiels der vorliegenden Erfindung.

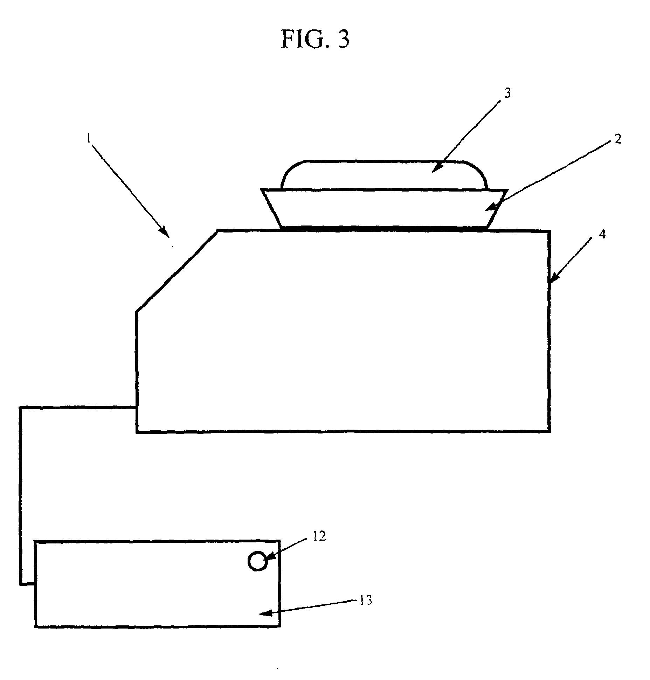

Fig. 3 zeigt eine schematische Darstellung des dritten Ausführungsbeispiels der vorliegenden Erfindung. Dieses dritte Ausführungsbeispiel der vorliegenden Erfindung ist ausgenommen der nachstehend dargelegten Unterschiede zu ihrem ersten Ausführungsbeispiel gleich. Aus diesem Grund sind in Fig. 3 nur die zu dem ersten Ausführungsbeispiel unterschiedlichen Bestandteile dargestellt.

Gemäß dem dritten Ausführungsbeispiel der vorliegenden Erfindung ist das als Spracheingabeeinrichtung dienende Mikrofon 12 nicht getrennt an das Blutentnahmegerät 1 gekoppelt, sondern ist in die Tastatur 13 eingebaut vorgesehen Damit kann die Bedienbarkeit des Blutentnahmegeräts 1 weiter vereinfacht werden. Alternativ kann das Mikrofon 12 an die Tastatur 13 gekoppelt sein.

Ausgenommen dieses Unterschieds gelten alle anderen bezüglich des ersten Ausführungsbeispiels dargelegten Erläuterungen ebenso für das dritte Ausführungsbeispiel.

Nachstehend erfolgt die Beschreibung eines vierten Ausführungsbeispiels der vorliegenden Erfindung.

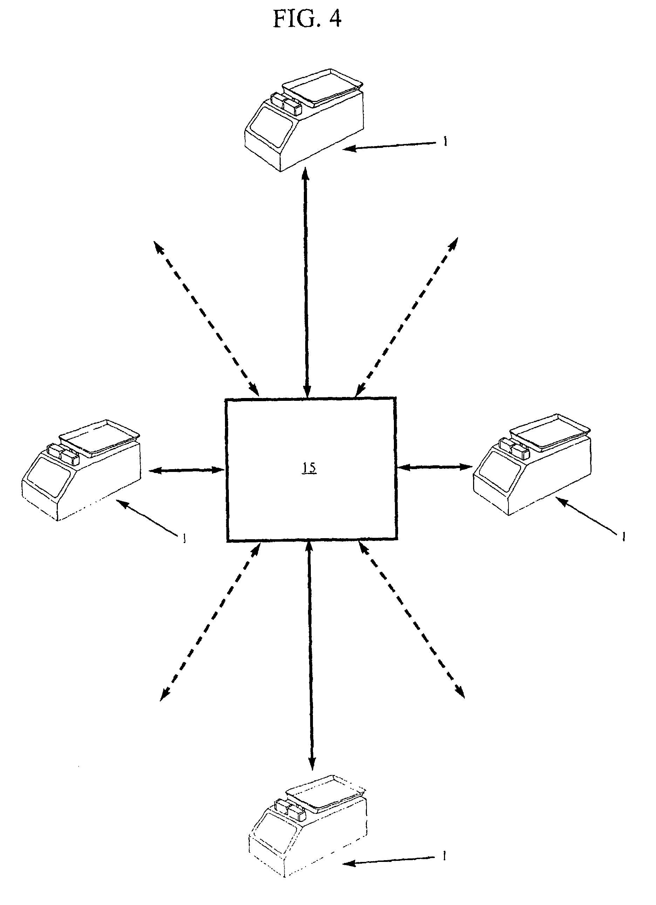

Fig. 4 zeigt schematisch ein System gemäß dem vierten Ausführungsbeispiel der vorliegenden Erfindung. Dieses System wird zum Erfassen und Verarbeiten von Daten verwendet, die eine Blutentnahme betreffen. Das System weist mindestens ein Blutentnahmegerät 1 nach einem der vorhergehenden Ausführungsbeispiele auf, wobei dieses mindestens eine Blutentnahmegerät 1 mit einer Übertragungseinrichtung, vorzugsweise einer drahtlosen Übertragungseinrichtung, ausgestattet ist. Weiterhin ist eine externe Verarbeitungseinheit 15 vorgesehen.

Diese externe Verarbeitungseinheit 15 weist eine Übertragungseinrichtung, eine Verarbeitungseinrichtung und eine Speichereinrichtung auf.

Die Übertragungseinrichtung dient zur wechselseitigen Übertragung von Daten zwischen dem mindestens einen Blutentnahmegerät 1 und der externen Verarbeitungseinheit 15. Vorzugsweise ist eine Mehrzahl von Blutentnahmegeräten 1 mit der externen Verarbeitungseinheit 15 gekoppelt, wie es schematisch in Fig. 5 mittels der vier Blutentnahmegeräte 1 und den gestrichelten Linien dargestellt ist. Die Daten der einzelnen Blutentnahmegeräte 1 werden zu der externen Verarbeitungseinheit 15 übertragen. In der Verarbeitungseinrichtung der externen Verarbeitungseinheit 15 werden die Daten zweckmäßig verarbeitet und zu der Speichereinrichtung ausgegeben, in der sie schließlich gespeichert werden.

Zur Unterscheidung der Daten der unterschiedlichen mit der externen Verarbeitungseinheit 15 gekoppelten Blutentnahmegeräte 1 können geläufige Verfahren verwendet werden, die deshalb hier nicht näher erläutert werden. Zum Beispiel kann jedes Blutentnahmegerät 1 eine für es kennzeichnende Kennung zu den zu übertragenden Daten hinzufügen und kann die externe Verarbeitungseinheit 15 die einzelnen Blutentnahmegeräte 1 zyklisch zur Datenübertragung auffordern.

Bezüglich noch weiterer, nicht näher erläuterter Wirkungen und Vorteile der Erfindung wird ausdrücklich auf die Offenbarung der Figuren verwiesen.

## Patentansprüche

1. Verwendung einer Spracheingabeeinrichtung (12) in einem Blutentnahmegerät zum Erfassen derjenigen Daten, die in für ein jeweiliges Land gültigen Richtlinien zur Blutentnahme festgelegt sind, wobei das Blutentnahmegerät aufweist:
eine Aufnahmevorrichtung (2) zum Aufnehmen eines Blutbeutels oder eines Blutentnahmegefäßes (3),
eine mit der Aufnahmevorrichtung (2) gekoppelte Wägeeinrichtung (5) zum Wägen eines Inhalts des Blutbeutels oder Blutentnahmegefäßes (3) und zum Ausgeben von Daten, die einem Inhalt des Blutbeutels oder Blutentnahmegefäßes (3) entsprechen,
eine Wandlereinrichtung (7) zum Umwandeln der mittels der Spracheingabeeinrichtung (12) eingegebenen Sprachdaten in Daten eines verarbeitbaren Datenformats, und
eine Erfassungseinrichtung (6) zum Erfassen mindestens der Daten aus der Wägeeinrichtung (5) und der Wandlereinrichtung (7).

2. Verwendung nach Anspruch 1, ***dadurch gekennzeichnet, daß*** die Spracheingabeeinrichtung (12) ein getrennt an das Blutentnahmegerät (1) angekoppeltes oder in das Blutentnahmegerät (1) eingebautes Mikrofon ist.

3. Verwendung nach Anspruch 1 oder 2, ***gekennzeichnet durch*** eine Tastatur (13) zum Eingeben von Daten, und/oder einen Strichcodeleser (14) zum Eingeben von Daten, wobei die Erfassungseinrichtung (6) die von der Tastatur (13) und/oder dem Strichcodeleser (14) eingegebenen Daten erfaßt.

4. Verwendung nach Anspruch 3, ***dadurch gekennzeichnet, daß*** die Spracheingabeeinrichtung (12) ein in die Tastatur (13) eingebautes oder an die Tastatur (13) gekoppeltes oder ein in den Strichcodeleser (14) eingebautes oder an den Strichcodeleser (14) angekoppeltes Mikrofon ist.

5. Verwendung nach einem der vorhergehenden Ansprüche, ***gekennzeichnet durch*** eine Ausgabeeinrichtung (8) zum Ausgeben der von der Spracheingabeeinrichtung (12) eingegebenen und von der Erfassungseinrichtung (6) erfaßten Daten.

6. Verwendung nach Anspruch 5, ***dadurch gekennzeichnet, daß*** die Ausgabeeinrichtung (8) eine Sprachausgabeeinrichtung und/oder eine Anzeigeeinrichtung aufweist.

7. Verwendung nach einem der vorhergehenden Ansprüche, ***gekennzeichnet durch*** eine Bewegungsvorrichtung zum schwenkenden Bewegen der Aufnahmevorrichtung (2).

8. Verwendung nach einem der vorhergehenden Ansprüche, ***gekennzeichnet durch*** eine Zufuhrstoppvorrichtung, die die Blutzufuhr in den Blutbeutel oder das Blutentnahmegefäß (3) bei Erreichen einer vorbestimmten Blutmenge unterbricht.

9. Verwendung nach einem der vorhergehenden Ansprüche, ***gekennzeichnet durch*** eine Verarbeitungseinrichtung (9) zum Verarbeiten der von der Erfassungseinrichtung (6) erfaßten Daten.

10. Verwendung nach Anspruch 9, ***gekennzeichnet durch*** eine Speichereinrichtung (10) zum Speichern der von der Verarbeitungseinrichtung (9) verarbeiteten Daten.

11. Verwendung nach Anspruch 10, ***dadurch gekennzeichnet, daß*** die Speichereinrichtung (10) abnehmbar ist.

12. Verwendung nach Anspruch 11, ***dadurch gekennzeichnet, daß*** die Speichereinrichtung (10) eine Speicherkarte ist.

13. Verwendung nach einem der vorhergehenden Ansprüche, ***gekennzeichnet durch*** eine Massenspeichereinrichtung, die innerhalb oder außerhalb des Blutentnahmegeräts (1) vorgesehen ist.

14. Verwendung nach Anspruch 13, ***dadurch gekennzeichnet, daß*** die Massenspeichereinrichtung ein CDROM-Laufwerk ist.

15. Verwendung nach einem der vorhergehenden Ansprüche, ***gekennzeichnet durch*** eine Übertragungseinrichtung (11) zum drahtgebundenen oder drahtlosen wechselseitigen Übertragen von Daten mit einer externen Verarbeitungseinheit.

16. Verwendung nach Anspruch 15, ***dadurch gekennzeichnet, daß*** die Übertragungseinrichtung (11) eine RS232-Schnittstelle, eine Funkschnittstelle oder eine Infrarotschnittstelle ist.

17. Verwendung einer Spracheingabeeinrichtung (12) nach einem der vorangehenden Ansprüche in einem System zum Erfassen und Verarbeiten von eine Blutspende betreffenden Daten, das mindestens ein Blutentnahmegerät (1) nach Anspruch 15 oder 16 und eine externe Verarbeitungseinheit (15) aufweist, wobei die externe Verarbeitungseinheit (15) weiterhin aufweist:
eine Übertragungseinrichtung zum wechselseitigen Übertragen von Daten mit dem mindestens einen Blutentnahmegerät (1);
eine Verarbeitungseinrichtung zum Verarbeiten der von dem mindestens einem Blutentnahmegerät (1) zu der zentralen Verarbeitungseinheit (15) übertragenen Daten; und
eine Speichereinrichtung zum Speichern der von der Verarbeitungseinrichtung verarbeiteten Daten.

## Claims

1. Use of a speech inputting device (12) in a blood sampling device for acquisition of those data which are stipulated in blood sampling guidelines applicable for a respective country, the blood sampling device comprising:
a reception device (2) to receive a blood bag or a blood sampling vessel (3),
a weighing device (5) coupled to the reception device (2) for weighing the contents of the blood bag or blood sampling vessel (3) and for outputting data corresponding to the contents of the blood bag or blood sampling vessel (3),
a converter device (7) for converting to data of a processable data format those speech data which have been input by means of the speech inputting device (12), and
an acquisition device (6) for acquisition at least of the data from the weighing device (5) and from the converter device (7).

2. Use according to claim 1, **characterised in that** the speech inputting device (12) is a microphone separately coupled to the blood sampling device (1) or incorporated in the blood sampling device (1).

3. Use according to claim 1 or 2, **characterised by** a keyboard (13) for inputting data and/or a barcode reader (14) for inputting data, the acquisition device (6) acquiring the data input from the keyboard (13) and/or the barcode reader (14).

4. Use according to claim 3, **characterised in that** the speech inputting device (12) is a microphone incorporated in the keyboard (13) or coupled to the keyboard (13) or incorporated in the barcode reader (14) or coupled to the barcode reader (14).

5. Use according to any one of the preceding claims, **characterised by** an outputting device (8) for outputting the data input by the speech inputting device (12) and acquired by the acquisition device (6).

6. Use according to claim 5, **characterised in that** the outputting device (8) comprises a speech outputting device and/or a display device.

7. Use according to any one of the preceding claims, **characterised by** a movement device for pivotal movement of the reception device (2).

8. Use according to any one of the preceding claims, **characterised by** a feed stopping device which stops the blood feed to the blood bag or the blood sampling vessel (3) when a predetermined quantity of blood has been reached.

9. Use according to any one of the preceding claims, **characterised by** a processing device (9) for processing the data acquired by the acquisition device (6).

10. Use according to claim 9, **characterised by** a memory device (10) for storage of the data processed by the processing device (9).

11. Use according to claim 10, **characterised in that** the memory device (10) is removable.

12. Use according to claim 11, **characterised in that** the memory device (10) is a memory card.

13. Use according to any one of the preceding claims, **characterised by** a bulk memory device which is provided inside or outside the blood sampling device (1).

14. Use according to claim 13, **characterised in that** the bulk memory device is a CD ROM drive.

15. Use according to any one of the preceding claims, **characterised by** a transmission device (11) for wired or wireless two-way exchange of data with an external processing unit.

16. Use according to claim 15, **characterised in that** the transmission device (11) is an RS 232 interface, a radio interface or an infrared interface.

17. Use of a speech inputting device (12) according to any one of the preceding claims in a system for the acquisition and processing of data relating to a blood sample, comprising at least one blood sampling device (1) according to claim 15 or 16 and an external processing unit (15), the external processing unit (15) also comprising:
a transmission device for the two-way exchange of data with the at least one blood sampling device (1);
a processing device for processing the data transmitted from the at least one blood sampling device (1) to the central processing unit (15); and
a memory device for storing the data processed by the processing device.

## Revendications

1. Utilisation de dispositif d'entrée vocale (12) dans un appareil de prélèvement de sang pour détecter les données, qui sont fixées dans des directives, valables pour un pays respectif, pour le prélèvement de sang, l'appareil de prélèvement de sang comprenant:
un dispositif de réception (2) pour recevoir un sachet de sang ou un récipient (3) de prélèvement du sang;
un dispositif de pesée (5) couplé au dispositif de réception (2) pour peser le contenu du sachet de sang ou du récipient (3) de prélèvement du sang et pour délivrer des données, qui correspondent au contenu du sachet de sang ou du récipient (3) de prélèvement de sang,
un convertisseur (7) servant à convertir les données vocales, introduites à l'aide du dispositif d'entrée vocale (12), en des données ayant un format de données pouvant être traité, et
un dispositif de détection (6) pour détecter au moins les données provenant du dispositif de pesée (5) et du convertisseur (7).

2. Utilisation selon la revendication 1, **caractérisée en ce que** le dispositif d'entrée vocale (12) est un microphone couplé séparément à l'appareil (1) de prélèvement de sang ou incorporé dans l'appareil (1) de prélèvement de sang.

3. Utilisation selon la revendication 1 ou 2, **caractérisée par** un clavier (13) pour l'introduction de données et/ou un lecteur de code à barres (14) pour introduire des données, le dispositif de détection (6) détectant les données introduites à partir du clavier (13) et/ou par le lecteur de codes à barres (14).

4. Utilisation selon la revendication 3, **caractérisée en ce que** le dispositif d'entrée vocale (12) est un microphone intégré dans le clavier (13) ou couplé au clavier (13) ou un microphone intégré dans le lecteur de codes à barres (14) ou couplé au lecteur de codes à barres (14).

5. Utilisation selon l'une des revendications précédentes, **caractérisée par** un dispositif de sortie (8) servant à délivrer les données introduites par le dispositif d'entrée vocale (12) et détectées par le dispositif de détection (6).

6. Utilisation selon la revendication 5, **caractérisée en ce que** le dispositif de sortie (6) comporte un dispositif de sortie vocale et/ou un dispositif d'affichage.

7. Utilisation selon l'une des revendications précédentes, **caractérisée par** un dispositif de déplacement servant à déplacer par pivotement le dispositif de réception (2).

8. Utilisation selon l'une des revendications précédentes, **caractérisée par** un dispositif d'arrêt d'amenée, qui interrompt l'arrivée de sang dans le sachet de sang ou dans le récipient (3) de prélèvement de sang, lorsqu'une quantité de sang prédéterminée est atteinte.

9. Utilisation selon l'une des revendications précédentes, **caractérisée par** un dispositif de traitement (9) pour traiter les données détectées par le dispositif de détection (6).

10. Utilisation selon la revendication 9, **caractérisée par** un dispositif de mémoire (10) pour mémoriser les données traitées par le dispositif de traitement (9).

11. Utilisation selon la revendication 10, **caractérisée en ce que** le dispositif de mémoire (10) est amovible.

12. Utilisation selon la revendication 11, **caractérisée en ce que** le dispositif de mémoire (10) est une carte de mémoire.

13. Utilisation selon l'une des revendications précédentes, **caractérisée par** le dispositif de mémoire de masse, qui est prévu à l'intérieur ou à l'extérieur de l'appareil (1) de prélèvement de sang.

14. Utilisation selon la revendication 13, **caractérisée en ce que** le dispositif de mémoire de masse est une unité CDROM.

15. Utilisation selon l'une des revendications précédentes, **caractérisée par** un dispositif de transmission (11) pour la transmission réciproque, à l'aide d'un câble ou sans câble, de données comportant une unité de traitement externe.

16. Utilisation selon la revendication 15, **caractérisée en ce que** le dispositif de transmission (11) est une interface RS232, une interface hertzienne ou une interface infrarouge.

17. Utilisation d'un dispositif d'entrée vocale (12) selon l'une des revendications précédentes, dans un système pour détecter et traiter des données concernant un don de sang, et qui comporte au moins un appareil (1) de prélèvement selon la revendication 15 ou 16 et une unité externe de traitement (15), l'unité externe de traitement (15) comportant en outre:
un dispositif de transmission pour la transmission réciproque de données comportant au moins un appareil (1) de prélèvement de sang;
un dispositif de traitement pour traiter les données transmises par le au moins un appareil (1) de prélèvement de sang à l'unité centrale de traitement (16); et
un dispositif de mémoire pour mémoriser les données traitées par le dispositif de traitement.
